(19)

Europäisches
Patentamt
European
Patent Office
Office européen
des brevets

(11) **EP 4 591 901 A1**

(12) **EUROPEAN PATENT APPLICATION**
published in accordance with Art. 153(4) EPC

(43) Date of publication:
**30.07.2025 Bulletin 2025/31**

(21) Application number: **23878649.5**

(22) Date of filing: **07.06.2023**

(51) International Patent Classification (IPC):
**A61M 5/168** $^{(2006.01)}$  **G16H 20/17** $^{(2018.01)}$
**A61M 5/172** $^{(2006.01)}$

(86) International application number:
**PCT/CN2023/099003**

(87) International publication number:
**WO 2024/082651 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.10.2022 CN 202211281716**

(71) Applicant: **Shandong Situring Co., Ltd.**
**Jinan, Shandong 250000 (CN)**

(72) Inventor: **PENG, Can**
**Jinan, Shandong 250000 (CN)**

(74) Representative: **Isern Patentes y Marcas S.L.**
**Avda. Diagonal, 463 Bis, 2°**
**08036 Barcelona (ES)**

(54) **ADAPTIVE CLOSED-LOOP CONTROL METHOD BASED ON LINEAR MODEL**

(57)     The present disclosure provides a linear model-based adaptive closed-loop control method, which is a control method to control a target physiological parameter of an object (2) by using a chemical substance, including: acquiring preset parameters (S101), acquiring the target physiological parameter of the object (2) and a residual activity parameter of the chemical substance (S103), calculating a sensitivity coefficient of the chemical substance based on the target physiological parameter and the residual activity parameter(S105), calculating an increase rate coefficient of the target physiological parameter based on the target physiological parameter and the residual activity parameter, the increase rate coefficient being used for characterizing an influence level of factors other than the chemical substance on the target physiological parameter(S107), calculating a safe dose and a basal dose of the chemical substance based on the preset parameters, the target physiological parameter, the residual activity parameter, the sensitivity coefficient and the increase rate coefficient (S109), and calculating a target dose of the chemical substance based on the safe dose and the basal dose (S111). In this case, a physiological parameter related to the target physiological parameter can be adaptively adjusted, and input parameters are reduced.

Fig. 1

**Description**

**BACKGROUND OF INVENTION**

**Field of Invention**

The present disclosure generally relates to the field of medical devices, and in particularly relates to a linear model-based adaptive closed-loop control method.

**Description of Related Art**

Diabetes is a metabolic disease characterized by hyperglycemia, for diabetes patients, they have symptoms such as hyperglycemia, unstable blood glucose and dyspepsia due to an insulin secretion defect and/or an impaired biological function. In this case, chronic damage will be brought to various kinds of tissue (especially eyes, kidney, heart, blood vessels and nerves). In some cases, the diabetes patients will be treated by taking medicines to relieve symptoms. For example, type 1 diabetes patients will be injected with insulin to avoid the hyperglycemia, and some type 1 diabetes patients will also use an insulin pump for adjuvant treatment, however, since an increase or decrease of blood glucose is related to various factors, it is difficult to accurately control a change of the blood glucose. For a treatment method of injecting the insulin, it is easy to cause continuous hyperglycemia due to insufficient injected volume or hypoglycemia due to excessive injected volume, thereby endangering the health of the diabetes patients. During medicine taking, in order to reduce untoward reactions caused by overdose or underdose of the medicine, the dose of the medicine will be regulated by using a closed-loop algorithm.

At present, commonly used closed-loop algorithms include a conventional PID algorithm or an action model algorithm. It is necessary for the conventional PID algorithm to input multiple parameters with uncertain physiological significance, and therefore, a personalized adjustment is required, while it is necessary for the action model algorithm to input intaking carbohydrate that is difficult to accurately quantify. For example, a Chinese patent application with a publication number of CN113453619A discloses a safety tool to make decision support recommendations for a user of a continuous glucose monitoring system, which requires to receive multiple input data terms that affect a diabetes condition of a user of a continuous glucose monitor. The multiple input data terms include nutrition data of a food or drink (equivalent to the intaking carbohydrate). Therefore, the current closed-loop algorithm for regulating the dose of the medicine still has a relatively high threshold for use, and the input of intaking carbohydrate that is difficult to accurately quantify is not conducive to daily use of an ordinary user.

**SUMMARY OF INVENTION**

The present disclosure is completed in view of a state of the prior art, and its purpose is to provide a linear model-based adaptive closed-loop control method that can reduce input parameters and meanwhile can adaptively adjust a physiological parameter related to a target physiological parameter.

For the above purpose, the present disclosure proposes a linear model-based adaptive closed-loop control method, which is a control method to control a target physiological parameter of an object by using a chemical substance, and includes: acquiring preset parameters, acquiring a target physiological parameter of the object and a residual activity parameter of the chemical substance, calculating a sensitivity coefficient of the chemical substance based on the target physiological parameter and the residual activity parameter, the sensitivity coefficient being used for characterizing an influence level of the chemical substance on the target physiological parameter, calculating an increase rate coefficient of the target physiological parameter based on the target physiological parameter and the residual activity parameter, the increase rate coefficient being used for characterizing an influence level of factors other than the chemical substance on the target physiological parameter, calculating a safe dose and basal dose of the chemical substance based on the preset parameters, the target physiological parameter, the residual activity parameter, the sensitivity coefficient and the increase rate coefficient, and calculating a target dose of the chemical substance based on the safe dose and the basal dose.

In this case, the sensitivity coefficient and the increase rate coefficient can be adaptively adjusted, which improves control accuracy. At the same time, since the increase rate coefficient can be used for characterizing the influence level of the factors other than the chemical substance on the target physiological parameter, and the increase rate coefficient is obtained by calculation of the target physiological parameter and the residual activity parameter, resulting in reducing input parameters (such as the carbohydrate intake of a subject), and then, lowering the threshold for use of the adaptive closed-loop control method.

In addition, in an adaptive closed-loop control method involved in an embodiment of the present disclosure, optionally, the preset parameters include a physiological information of the object, a type of the chemical substance, a control step size, an unit of the target physiological parameter, a safety threshold value of the target physiological parameter, and an infusion accuracy of the chemical substance. In this case, the parameters can be adaptively adjusted in advance according to the preset parameters, which improves the control accuracy.

In addition, in an adaptive closed-loop control method involved in an embodiment of the present disclosure, optionally, the preset parameters are obtained by means of data import or manual input. In this case, the preset parameters can be conveniently obtained by means of data import, which further lowers a threshold for use. The preset parameters can be conveniently modified by means of manual input.

In addition, in an adaptive closed-loop control method involved in an embodiment of the present disclosure, optionally, the chemical substance of the infusion dose is enabled to enter the object by means of infusion. The infusion dose is related to the target dose and the infusion accuracy. In this case, the calculated target dose can cooperate with actual infusion accuracy of an actual device to obtain the infusion dose that can be infused to the target within an accuracy range.

In addition, in an adaptive closed-loop control method involved in an embodiment of the present disclosure, optionally, the infusion dose is obtained by metering of an infusing device. In this case, due to uncertainty of actual infusion, the infusion dose is obtained by using metering of the infusing device, so that the actual dose of the chemical substance infused into the object can be more accurately obtained, it is facilitated to improve the accuracy of subsequent regulation.

In addition, in an adaptive closed-loop control method involved in an embodiment of the present disclosure, optionally, the target physiological parameter is collected by a continuous blood glucose monitoring apparatus or a blood glucose meter. In this case, the target physiological parameter can be acquired in different ways.

In addition, in an adaptive closed-loop control method involved in an embodiment of the present disclosure, optionally, the target physiological parameters of the object at a plurality of time nodes and the residual activity parameter of the chemical substance are acquired, the plurality of time nodes include a target node and other nodes, and the residual activity parameter of the target node is obtained based on the residual activity parameters of the other nodes. In this case, parameters such as the residual activity parameter, the increase rate coefficient, or the sensitivity coefficient will not change too much in a short time. When parameters such as a residual activity parameter, an increase rate coefficient, or a sensitivity coefficient of one time node are calculated, the accuracy of calculation can be improved by using parameters of a plurality of time nodes.

In addition, in an adaptive closed-loop control method involved in an embodiment of the present disclosure, optionally, the sensitivity coefficient located at the target node is obtained by minimizing a loss function. The loss function includes an error term and a regularization term. The error term is used for characterizing a difference between the target physiological parameter acquired by measurement and the target physiological parameter acquired by calculation. The regularization term is used for characterizing a difference degree of the sensitivity coefficients of adjacent time nodes. In this case, the accuracy of the sensitivity coefficient of each time node can be improved by using an error term. At the same time, since the sensitivity coefficient will not change too much in a short time under a normal circumstance, the stability and accuracy of the sensitivity coefficient can also be improved by using the regularization term.

In addition, in an adaptive closed-loop control method involved in an embodiment of the present disclosure, optionally, the sensitivity coefficient of the target node is acquired based on the sensitivity coefficients of the other nodes, the target physiological parameters of the other nodes, the residual activity parameters of the other nodes, and the target physiological parameter of the target node. In this case, the sensitivity coefficient of the target node can be calculated by using some of parameters acquired in previous time nodes, thereby adaptively adjusting the sensitivity coefficient. At the same time, since the sensitivity coefficient will not change too much in the adjacent time nodes, the stability and accuracy of the sensitivity coefficient can be improved by using the adjacent time nodes.

In addition, in an adaptive closed-loop control method involved in an embodiment of the present disclosure, optionally, the increase rate coefficient of the target node is obtained based on the difference between the target physiological parameter acquired by measurement and the target physiological parameter acquired by calculation located at the target node, and the increase rate coefficients of the other nodes. In this case, the increase rate coefficient of the target node can be calculated by using some of parameters acquired in previous time nodes, thereby adaptively adjusting the increase rate coefficient, so that the accuracy of the sensitivity coefficient can be improved.

According to the present disclosure, a linear model-based adaptive closed-loop control method that can reduce input parameters and meanwhile can adaptively adjust a physiological parameter related to a target physiological parameter can be provided.

## BRIEF DESCRIPTION OF THE DRAWINGS

The embodiments of the present disclosure will now be further explained in detail only by referring to the examples in the accompanying drawings, wherein:

Fig. 1 is a schematic diagram illustrating an application scenario of a linear model-based adaptive closed-loop control method involved in an embodiment of the present disclosure.

Fig. 2 is a schematic diagram illustrating a two compartment dynamics model involved in an embodiment of the present disclosure.

Fig. 3 is a flow chart illustrating a linear model-based adaptive closed-loop control method involved in an embodiment of the present disclosure.

Fig. 4 is a schematic diagram illustrating an application scenario of acquiring a target physiological parameter of an object involved in an embodiment of the present disclosure.

Fig. 5 is a curve diagram illustrating a target physiological parameter involved in an embodiment of the present disclosure.

Fig. 6 is a structural block diagram illustrating a linear model-based adaptive closed-loop control system involved in an embodiment of the present disclosure.

Fig. 7 is a flow chart illustrating a linear model-based adaptive closed-loop control system involved in an embodiment of the present disclosure.

Fig. 8a is a simulation schematic diagram illustrating that a plain PID method is applied to an adult involved in an embodiment of the present disclosure.

Fig. 8b is a simulation schematic diagram illustrating that a PIDIFB method is applied to an adult involved in an embodiment of the present disclosure.

Fig. 8c is a simulation schematic diagram illustrating that an adaptive closed-loop control method is applied to an adult involved in an embodiment of the present disclosure.

Fig. 9a is a simulation schematic diagram illustrating that a plain PID method is applied to an adolescent involved in an embodiment of the present disclosure.

Fig. 9b is a simulation schematic diagram illustrating that a PIDIFB method is applied to an adolescent involved in an embodiment of the present disclosure.

Fig. 9c is a simulation schematic diagram illustrating that an adaptive closed-loop control method is applied to an adolescent involved in an embodiment of the present disclosure.

Fig. 10a is a simulation schematic diagram illustrating that a plain PID method is applied to a child involved in an embodiment of the present disclosure.

Fig. 10b is a simulation schematic diagram illustrating that a PIDIFB method is applied to a child involved in an embodiment of the present disclosure.

Fig. 10c is a simulation schematic diagram illustrating that an adaptive closed-loop control method is applied to a child involved in an embodiment of the present disclosure.

Fig. 11 is a simulation schematic diagram illustrating that an optimized adaptive closed-loop control method is applied to a child involved in an embodiment of the present disclosure.

Fig. 12a is a simulation schematic diagram illustrating that an adaptive closed-loop control method with infinite infusion accuracy is applied to an adult involved in an embodiment of the present disclosure.

Fig. 12b is a simulation schematic diagram illustrating that an adaptive closed-loop control method with finite infusion accuracy is applied to an adult involved in an embodiment of the present disclosure.

## DETAILED DESCRIPTION

Hereinafter, the preferred embodiments of the present disclosure will be described in detail with reference to the accompanying drawings. In the following description, the same symbols are assigned to the same components, and the

# EP 4 591 901 A1

repeated description is omitted. In addition, the drawings are only schematic drawings, and the proportions of the dimensions between components or the shapes of the components may be different from the actual ones.

It should be noted that the terms "include" and "have" in the present disclosure and any variations thereof. For example, the processes, methods, systems, products or devices of a series of steps or units that the present disclosure includes are unnecessarily limited to those steps or units that are clearly listed, but may include or have other steps or units that are not clearly listed or are inherent to these processes, methods, products or devices.

An embodiment of the present disclosure relates to a linear model-based adaptive closed-loop control method, which represents and characterizes an influence level of factors other than a chemical substance on a target physiological parameter by using an increase rate coefficient, and meanwhile, adaptively adjusts a sensitivity coefficient and the increase rate coefficient. In this case, a physiological parameter related to the target physiological parameter can be adaptively adjusted, and input parameters are reduced.

An embodiment of the present disclosure relates to a linear model-based adaptive closed-loop control system, which control a target physiological parameter of an object by using the adaptive closed-loop control method involved in the embodiment of the present disclosure. In this case, a physiological parameter related to the target physiological parameter can be adaptively adjusted, the stability of the target physiological parameter of the object is improved, and input parameters are reduced.

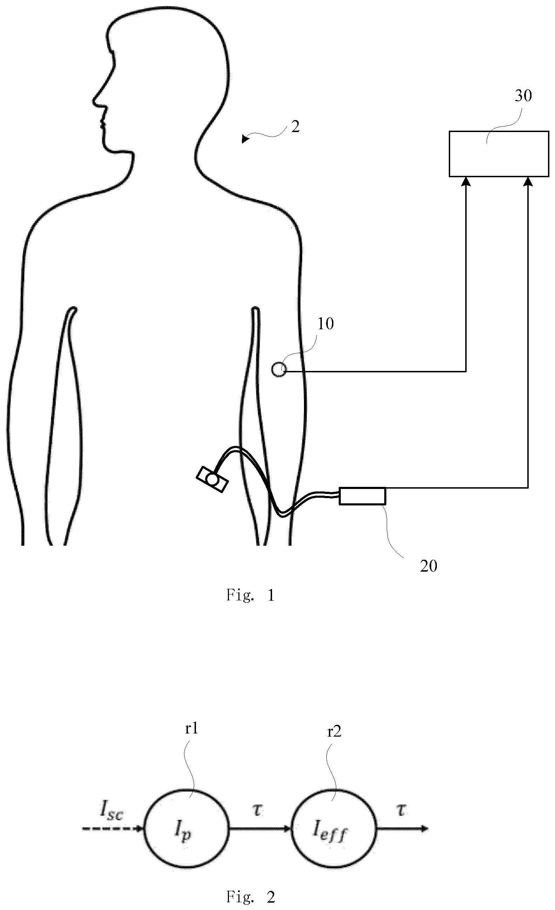

Fig. 1 is a schematic diagram illustrating an application scenario of a linear model-based adaptive closed-loop control method involved in an embodiment of the present disclosure.

In some examples, referring to Fig. 1, the linear model-based adaptive closed-loop control method may be a control method to control a target physiological parameter of an object 2. In some examples, the linear model-based adaptive closed-loop control method involved herein may be referred as an LMPID method, a control method, or an adaptive closed-loop control method.

In some examples, an infusion dose of the chemical substance can be obtained based on the adaptive closed-loop control method, and the chemical substance is infused to an object 2 by an infusing device 20. However, the present disclosure is not limited to this, the chemical substance can also enter the object 2 in multiple ways. For example, according to constituents of the chemical substance, the chemical substance can also enter the object 2 by oral administration, sublingual administration, rectal administration, mucocutaneous administration, inhalation administration, injection administration, etc. Preferably, the chemical substance can be infused to the object 2 by the infusing device 20. In this case, the chemical substance can be delivered to the object 2 rapidly, and the take-effect time of the chemical substance is accelerated.

In some examples, the infusing device 20 may be an infusing device 20 fixed to the object 2, and automatic infusion is performed based on the infusion dose of the chemical substance acquired by the adaptive closed-loop control method. In some examples, the infusing device 20 may also be a needle tubing. The chemical substance can be manually infused into the object 2 or other patients based on the infusion dose of the chemical substance acquired by the adaptive closed-loop control method. Preferably, the infusing device 20 may be an insulin pump (or an artificial pancreas). In this case, the chemical substance (e.g., insulin) can be conveniently delivered, and meanwhile enabling to be combined with a continuous glucose monitoring apparatus 10a (CGM) and a processing apparatus 30 to form a closed-loop insulin pump system.

In some examples, the object 2 may be an animal. For example, the object 2 may be a creature such as a human, an orangutan or a mouse. In some examples, the object 2 may be a patient with an endocrine disease. For example, the object 2 may be a patient with a defective endocrine gland (adrenal gland, thyroid gland, pancreas or pituitary gland). In this case, since the patient with the endocrine disease may have an abnormal secretory function and/or structure of the endocrine gland or endocrine tissue itself, an imbalance of a chemical substance used for regulation in the body occurs, thus leading to an imbalance of the target physiological parameter. The chemical substance can be controlled by using the adaptive closed-loop control method involved in the embodiment of the present disclosure, and then, the target physiological parameter can be controlled within an appropriate range.

In some examples, the object 2 may be a patient with diabetes, hypopituitarism, a thyroid disease, or obesity. Preferably, the object 2 may be a patient with the diabetes, particularly type 1 diabetes (insulin dependent diabetes) patients. In this case, since the insulin is difficult to produce in the type 1 diabetes patient, hyperglycemia or large blood glucose fluctuation can be easily caused. The blood glucose can be effectively stabilized by using the adaptive closed-loop control method involved in the embodiment of the present disclosure.

In some examples, the chemical substance may be a substance with a special effect on the body that is directly secreted into blood by human and animal endocrine organs or tissue. For example, the chemical substance may be a hormone (including: the chemical substance may be a pituitary hormone, insulin, glucagon, calcitonin, a parathyroid hormone, etc.), preferably, the chemical substance may be insulin.

In some examples, the chemical substance may be a synthetic hormone. In some examples, the chemical substance may be a substance that have a regulation effect on the blood glucose.

In some examples, the target physiological parameter may refer to a blood glucose concentration of the object 2.

In some examples, the target physiological parameter may also refer to the substance in the blood that has the special effect on the body. For example, the target physiological parameters may also be some of hormones related to target physiological health.

Hereafter, in order to better describe the adaptive closed-loop control method, the adaptive closed-loop control method involved in the embodiment of the present disclosure is described by taking the object 2 as the diabetes patient, the chemical substance as insulin, and the target physiological parameter as the blood glucose concentration as an example. However, it should be noted that the adaptive closed-loop control system involved in the embodiment of the present disclosure is not limited to this. The adaptive closed-loop control system involved in the embodiment of the present disclosure is also applicable to other cases where the target physiological parameter of the object 2 are controlled by using the chemical substance.

Fig. 2 is a schematic diagram illustrating a two compartment dynamics model involved in an embodiment of the present disclosure.

In some examples, the adaptive closed-loop control method may be based on a blood glucose dynamics model. In some examples, the blood glucose dynamics model may be expressed as:

$$\Delta G(k+1) = G(k+1) - G(k) = \varphi I_{e\!f\!f} + \alpha$$

Wherein $\Delta G(k + 1)$ represents a change of the target physiological parameter at a k+1$^{th}$ time node (i.e., the change of the blood glucose concentration), $G(k + 1)$ represents the target physiological parameter at the k+1$^{th}$ time node (i.e., the blood glucose concentration), $\varphi$ represents the sensitivity coefficient (i.e., an insulin sensitivity coefficient) of the chemical substance, $I_{e\!f\!f}$ represents the residual activity parameter of the chemical substance (i.e., active insulin), and $\alpha$ represents the increase rate coefficient of the target physiological parameter.

In some examples, the blood glucose dynamics model is a linear model, so that the adaptive closed-loop control method may also be called the linear model-based adaptive closed-loop control method. In this case, since the linear model can conveniently reflect a rule of the blood glucose dynamics model at the adjacent time nodes (a short time interval), the calculation cost can be reduced, and the calculation speed is increased, and thus the timeliness of the adaptive closed-loop control method can be improved.

In some examples, a metabolic model of the insulin in the object 2 can be established by using a two-compartment model. In some examples, referring to Fig. 2, the two-compartment model may include a central compartment r1 and an outer peripheral compartment r2. Wherein the insulin in the central compartment r1 may be insulin that is infused intravenously and enters the compartment. The insulin in the outer peripheral compartment r2 may be insulin that diffuses from the compartment into blood vessels, which may also be known as the active insulin.

In some examples, the two-compartment model may be expressed as:

$$\frac{dI_p}{dt} = I_{sc} - \frac{I_p}{\tau},$$

$$\frac{dI_{e\!f\!f}}{dt} = \frac{I_p}{\tau} - \frac{I_{e\!f\!f}}{\tau},$$

$$h(t) = \frac{1}{\tau^2} te^{-\frac{t}{\tau}},$$

Wherein t may represent time, $I_p$ may represent the amount of the insulin in the central compartment r1, $I_{sc}$ may represent the infusion dose of the insulin (i.e., the insulin infused to the object), $\tau$ may represent time that insulin reaches a peak, and h (t) may represent an impact response of the system.

In some examples, based on the two-compartment model, the metabolic model may be expressed as:

$$I_{e\!f\!f}(k+1) = K_0 I_{sc}(k) + K_1 I_{e\!f\!f}(k) - K_2 I_{e\!f\!f}(k-1),$$

$$K_1 = 2e^{-\frac{\Delta t}{\tau}},$$

$$K_2 = e^{-\frac{2\Delta t}{\tau}},$$

$$K_0 = 1 - K_1 + K_2,$$

Wherein k may represent a $k^{th}$ time node, $\triangle t$ may represent a time interval between two adjacent time nodes, $\triangle t$ may also be called a control step size, and $K_0$, $K_1$ and $K_2$ may represent parameters related to diffusion rates of the insulin at different positions.

In some examples, $K_0$, $K_1$ and $K_2$ may be constants obtained on basis of the control step size and the time that the insulin reaches the peak, in other words, $K_0$, $K_1$ and $K_2$ can be obtained by the preset parameters.

In some examples, as described above, the adaptive closed-loop control method can be established based on the blood glucose dynamics model and the metabolic model.

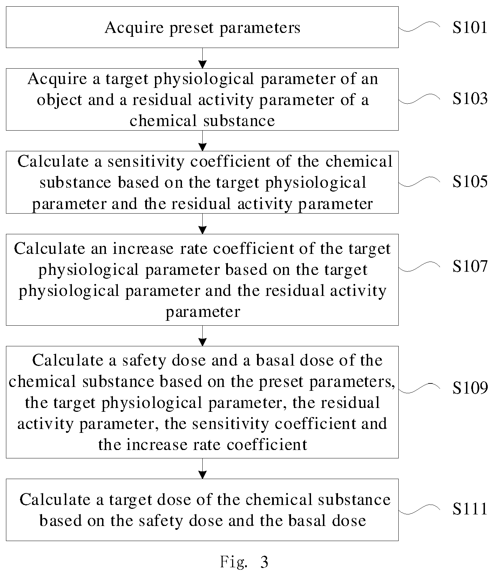

Fig. 3 is a flow chart illustrating a linear model-based adaptive closed-loop control method involved in an embodiment of the present disclosure.

In some examples, referring to Fig. 3, the adaptive closed-loop control method may include: acquiring the preset parameters (step S101), acquiring the target physiological parameter of the object 2 and residual activity parameter of the chemical substance (step S103), calculating a sensitivity coefficient of the chemical substance based on the target physiological parameter and the residual activity parameter (step S105), calculating an increase rate coefficient of the target physiological parameter based on the target physiological parameter and the residual activity parameter (step S107), calculating a safe dose and a basal dose of the chemical substance based on the preset parameters, the target physiological parameter, the residual activity parameter, the sensitivity coefficient and the increase rate coefficient (step S109), and calculating a target dose of the chemical substance based on the safe dose and the basal dose (step S111).

In this case, the sensitivity coefficient and the increase rate coefficient can be adaptively adjusted, which improves control accuracy. At the same time, since the increase rate coefficient can be used for characterizing the influence level of the factors other than the chemical substance on the target physiological parameter, and the increase rate coefficient is obtained by calculation of the target physiological parameter and the residual activity parameter, thereby reducing input parameters (such as the carbohydrate intake of the object 2), and then, the threshold for use of the adaptive closed-loop control method is lowered.

(Step S101)

In some examples, as described above, in step S101, the preset parameters may be acquired.

In some examples, the preset parameters may include at least one of the physiological information of the object 2, the type of the chemical substance, the control step size, the unit of the target physiological parameter, the safety threshold value of the target physiological parameter, and the infusion accuracy of the chemical substance. In this case, the parameters can be adaptively adjusted in advance according to the preset parameters, which improves the control accuracy.

In some examples, the preset parameters may include the physiological information of the object 2. In some examples, the physiological information may include gender, age, weight, course of disease and other easy-to-acquire medical information. In some examples, the physiological information may include a family history and a medication history. In this case, since the sensitivity coefficient of the chemical substance in different objects 2 is highly correlated with the personal physiological information of the object 2, an initial value of the sensitivity coefficient can be estimated by acquiring the physiological information.

In some examples, the preset parameters may include the type of the chemical substance. For example, the preset parameters may include the type of the insulin. In this case, since different types of chemical substances have different time to reach the peak in the blood, it is able to set the time for the chemical substance to reach the peak in the blood based on the type of the chemical substance.

In some examples, the preset parameters may include the control step size, which can be an interval between two adjacent time nodes. In this case, the control step size is input, which can adjust the time node interval, so that different control step sizes can be selected for different objects 2. For example, if a fluctuation of the target physiological parameter of the object 2 is relatively stable, a larger control step size can be selected as the control step size. If a fluctuation of the target physiological parameter of the object 2 is relatively severe, a shorter control step size can be selected as the control step size. In some examples, an appropriate control step size can also be selected for other reasons.

In some examples, the preset parameters may include the unit of the target physiological parameter. For example, in a case where the target physiological parameter is the blood glucose concentration, the target physiological parameter may be millimole per liter (mmol/l) or milligram per deciliter (mg/dl). In this case, due to different usage habits, people in different regions may use different units. Therefore, the unit of the target physiological parameter is input, which can facilitate the usage habits of different people, and reduces the difficulty of conversion, so that the versatility of the adaptive closed-loop control method can be improved.

In some examples, the preset parameters may include the safety threshold value of the target physiological parameter. In this case, when the infusion dose of chemical substance is acquired subsequently, the appropriate infusion dose of the chemical substance can be obtained based on the safety threshold value, so that the target physiological parameter can be controlled within a safety range.

In some examples, the preset parameters may include the infusion accuracy of the chemical substance. In this case, since different infusing devices 20 may have different infusion accuracy, a same infusing device 20 may be also set to have different infusion accuracy, and the dose of the chemical substance actually infused into the object 2 is related to the infusion accuracy of the infusing device 20, which may not be exactly the same as the target dose calculated by the adaptive closed-loop control method. By acquiring the infusion accuracy of the chemical substance, the accuracy of the chemical substance actually infused into the object 2 can be determined, and then the dose of the chemical substance actually infused into the object 2 can be better determined, thus facilitating the calculation of the adaptive closed-loop control method.

In some examples, the preset parameters are obtained by means of data import or manual input. For example, a part of the preset parameters can be obtained by data import, and a part of the preset parameters can be obtained by manual input. In this case, the preset parameters can be conveniently obtained by means of data import, which further lowers a threshold for use. The preset parameters can be conveniently modified by means of manual input.

In some examples, some of the preset parameters can be input by medical personnel. In this case, the medical personnel can set the preset parameters based on professional knowledge and experience. In some examples, the preset parameters can also be input by the object 2. In this case, the object 2 can adaptively adjust the preset parameters based on an individual condition.

In some examples, some of the preset parameters can be obtained through internal parameters of the infusing device 20, for example, by calling the internal parameters of the infusing device 20, or through the technical specifications or an instruction for use of the device.

(Step S103)

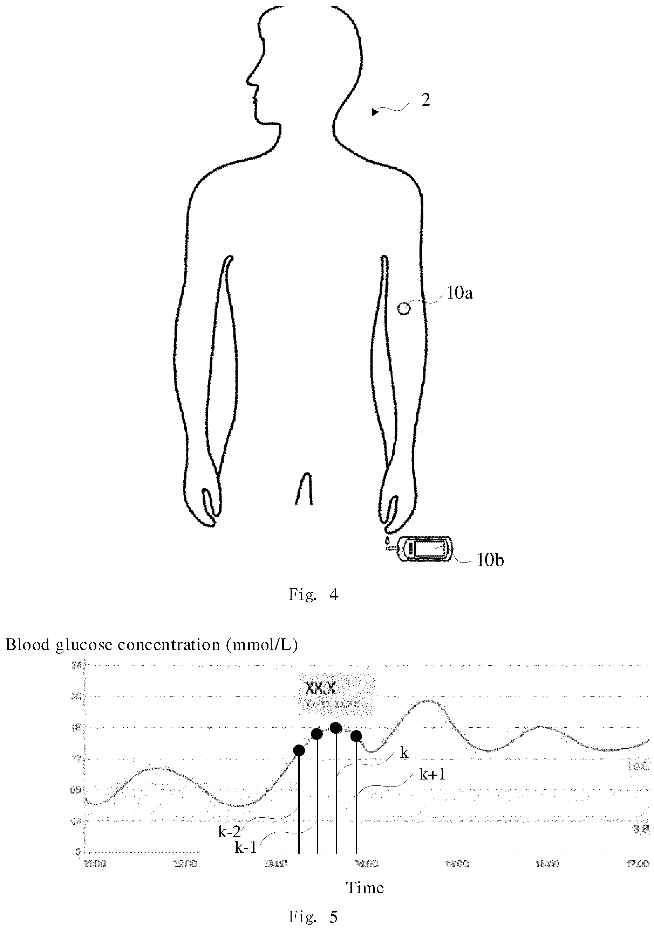

Fig. 4 is a schematic diagram illustrating an application scenario of acquiring a target physiological parameter of an object 2 involved in an embodiment of the present disclosure. Fig. 5 is a curve diagram illustrating a target physiological parameter involved in an embodiment of the present disclosure.

In some examples, step S103 may be acquiring the target physiological parameter of the object 2 and the residual activity parameter of the chemical substance. In this case, it is able to determine the residual activity parameter of the chemical substance. Since there may be the chemical substance still remaining active in the object 2, when the target dose is acquired by using the adaptive closed-loop control method, the residual activity parameter of the chemical substance can be taken into consideration, which can reduce the situation that the target physiological parameter exceeds the safety range due to an excessive target dose (for example, the blood glucose concentration is lower than the safety threshold value).

In some examples, the residual activity parameter of the chemical substance may be understood in the following way: the residual activity parameter is the amount (or concentration) of the chemical substance remaining active in the object 2, or the amount (or concentration) of the chemical substance that can still take effect and adjust the target physiological parameter.

In some examples, referring to Fig. 4, the target physiological parameter can be obtained by the continuous glucose monitoring apparatus 10a (CGM) or a blood glucose meter 10b. In other words, an acquiring device 10 may be the continuous glucose monitoring device 10a or the blood glucose meter 10b. In this case, the target physiological parameter can be acquired in various ways.

In some examples, the target physiological parameter can be continuously acquired by using the continuous blood glucose monitoring apparatus 10a. In this case, the continuous blood glucose monitoring apparatus 10a is used, which can improve the convenience of acquiring the target physiological parameter, and meanwhile it is facilitated for the processing apparatus 30 to process a mass of target physiological parameters. In some examples the continuous blood glucose monitoring apparatus 10a may be provided at a position such as an arm, abdomen or thigh.

In some examples, the blood of the object 2 can also be collected through a blood collection needle (for example, the blood is collected from a fingertip, palm, or arm), and a target physiological parameter in the finger blood can be measured using the blood glucose meter 10b. Preferably, the blood (finger blood) of the fingertip of the object 2 can be collected. In this case, the fingertip has a highly dense capillary network, the change of blood glucose concentration in the body can be quickly reflected, thus facilitating control of the blood glucose concentration.

In some examples, referring to Fig. 5, the target physiological parameter of the object 2 at a plurality of time nodes and the residual activity parameter of the chemical substance can be acquired. The plurality of time nodes may be continuous time nodes. In some examples, the plurality of time nodes include a target node and other nodes, and residual activity parameter of the target node are obtained based on residual activity parameters of the other nodes. In this case, parameters such as the residual activity parameter, the increase rate coefficient, or the sensitivity coefficient will not change too much in a short time. When parameters such as a residual activity parameter, an increase rate coefficient, or a sensitivity coefficient of one time node are calculated, the accuracy of calculation can be improved by using parameters of multiple time nodes.

In some examples, referring to Fig. 5, other nodes may be nodes before the target node. For example, the target node may be a k+1th time node, and the other nodes may be a kth time node and a k-1th time node. It should be noted that an identical time node may be a target node or one of other nodes. Specifically, one time node is allowed to correspond to one control step. In one control step, a fifth time node may be the target node, and a fourth time node and a third time node may be the other nodes. In a next control step, a sixth time node may be the target node, and a fifth time node and a fourth time node may be the other nodes.

In some examples, in one control step, the time nodes may include not less than two other nodes and one target node. Preferably, in one control step, the time nodes may include two other nodes and one target node. In this case, a large number of other nodes can reflect a complex algorithm model. A large number of other nodes can reduce the complexity of the algorithm and reduce the calculation cost.

In some examples, the residual activity parameter may satisfy the metabolic model described above:

$$I_{eff}(k+1) = K_0 I_{sc}(k) + K_1 I_{eff}(k) - K_2 I_{eff}(k-1) \,,$$

In this case, the active insulin of the target node can be obtained based on the insulin infusion dose of the insulin, and the active insulin of the other nodes.

(Step S105)

In some examples, step S105 may be calculating the sensitivity coefficient of the chemical substance based on the target physiological parameter and the residual activity parameter.

In some examples, the sensitivity coefficient may be used for characterizing the influence level of the chemical substance on the target physiological parameter. In a case where the chemical substance is the insulin, the sensitivity coefficient may also be called the insulin sensitivity coefficient. In some examples, the sensitivity coefficient is a negative. The smaller the sensitivity coefficient is, the greater the influence level of the chemical substance on the target physiological parameter is. The greater the sensitivity coefficient (closer to 0) is, the smaller the influence level of the chemical substance on the target physiological parameter is. In other words, the greater the absolute value of the sensitivity coefficient is, the greater the influence level of the chemical substance on the target physiological parameter is. The smaller the absolute value of the sensitivity coefficient is, the smaller the influence level of the chemical substance on the target physiological parameter is.

In some examples, the sensitivity coefficient may be obtained through formula iteration. In this case, the sensitivity coefficient at each time node can be obtained using formula iteration so as to obtain an adaptively adjusted sensitivity coefficient, resulting in improving the control accuracy.

In some examples, the initial value of the sensitivity coefficient may be actively input or calculated by the processing apparatus 30 based on the preset parameters. In some examples, the initial value of the sensitivity coefficient may be obtained through the ways such as rule 500, rule 1500 or rule 1800. In this case, the initial value of the sensitivity coefficient can be conveniently obtained, and then the sensitivity coefficient at each time node can be obtained by using the formula iteration.

In some examples, a calculation formula of the sensitivity coefficient may be obtained through loss functions. Specifically, the sensitivity coefficient located at the target node may be obtained through the way of minimizing loss function. The loss function includes an error term and a regularization term. The error term is used for characterizing a difference between the target physiological parameter acquired by measurement and the target physiological parameter acquired by calculation. The regularization term is used for characterizing a difference degree of the sensitivity coefficients of adjacent time nodes. In this case, the accuracy of the sensitivity coefficient of each time node can be improved by using an error term. Meanwhile, since the sensitivity coefficient will not change too much in a short time under a normal circumstance, the stability and accuracy of the sensitivity coefficient can also be improved by using the regularization term.

In some examples, the sensitivity coefficient of the target node may be acquired based on the sensitivity coefficients of the other nodes, the target physiological parameters of the other nodes, the residual activity parameters of the other nodes, and the target physiological parameter of the target node. In this case, the sensitivity coefficient of the target node can be calculated by using some of parameters acquired in a previous time node, thereby realize adaptive adjustment of the sensitivity coefficient. Meanwhile, since the sensitivity coefficient will not change too much in the adjacent time nodes, the stability and accuracy of the sensitivity coefficient can be improved by using the adjacent time nodes.

Hereinafter the acquisition way of the sensitivity coefficient is further explained. In some examples, the loss function may satisfy the formula:

$$L(\varphi) = \left| G(k) - G(k-1) - \alpha(k-1) - \varphi(k)I_{eff}(k-1) \right|^2 + \mu(\varphi(k) - \hat{\varphi}(k-1))^2$$

Wherein $L(\varphi)$ may represent the loss function, $G(k)$ may represent the target physiological parameter (i.e., the blood glucose concentration) at the $k^{th}$ time node, $\alpha(k-1)$ may represent an increase rate coefficient at the k-1$^{th}$ time node, $\varphi(k)$ may represent the sensitivity coefficient of the chemical substance at the $k^{th}$ time node (i.e., the insulin sensitivity coefficient), $I_{eff}(k-1)$ may represent the residual activity parameter (i.e., the active insulin) of the chemical substance at the k-1$^{th}$ time node, $\mu$ may represent a regularization term coefficient, which is used for adjusting an action ratio of the error term and the regularization term, $\hat{\varphi}(k-1)$ may represent a predicted value of the sensitivity coefficient of the chemical substance at the k-1$^{th}$ time node (i.e., the predicted value of the insulin sensitivity coefficient), $|G(k)-G(k-1)-\alpha(k-1)-\varphi(k)I_{eff}(k-1)|^2$ may represent the error term and $\mu(\varphi(k) - \hat{\varphi}(k-1))2$ may represent the regularization term.

It should be noted that the error term and the form of the error term are not limited to this, an expression that can be used for characterizing a difference between the target physiological parameter acquired by measurement and the target physiological parameter acquired by calculation can be used as the error term, for example, expressions for a ratio, a difference value or an absolute value (or a plurality of powers such as square and cube) of the difference value between the target physiological parameter acquired by measurement and the target physiological parameter acquired by calculation can be used as the error term, while expressions that can be used for characterizing a degree of difference of the sensitivity coefficients of adjacent time nodes can also be used as the regularization term, for example, expressions for a ratio, a difference value or an absolute value (or a plurality of powers such as square and cube) of the difference value between the sensitivity coefficients of the adjacent time nodes may be taken as the regularization term.

In some examples, the regularization term coefficients may be set empirically or may be default data set by the processing apparatus 30.

In some examples, the predicted value of the sensitivity coefficient of chemical substance may satisfy the formula:

$$\hat{\varphi}(k) = \arg\min(L(\varphi)),$$

Wherein arg min($L(\varphi)$) represents that $L(\varphi)$ is allowed to be minimized.

In some examples, a first iteration formula of the sensitivity coefficient may be obtained based on the formula satisfied by the predicted value of the sensitivity coefficient of the chemical substance. In some examples, the first iteration formula of the sensitivity coefficient may be obtained by derivation of $L(\varphi)$. In some examples, an iteration step size in the first iteration formula may be set empirically.

In some examples, the sensitivity coefficient acquired by calculation (i.e., the predicted value of the sensitivity coefficient) may be taken as sensitivity coefficients of other steps for calculation.

(Step S107)

In some examples, step S107 may be calculating the increase rate coefficient of the target physiological parameter based on the target physiological parameter and the residual activity parameter. In some examples, the increase rate coefficient may be used for characterizing the influence level of factors other than the chemical substance on the target physiological parameter. When the chemical substance is the insulin, the increase rate coefficient may also be called a blood glucose increase rate. The increase rate coefficient may be used for characterizing an influence level of factors such as carbohydrate intake, endogenous glucose secretion, or an unexplained change in insulin sensitivity on the blood glucose concentration. In this case, since the increase rate coefficient can be used to reflect effects of various factors, and the calculation of the increase rate coefficient does not require the input of carbohydrate (described later), the input of the parameters can be reduced, and meanwhile, the accuracy of the model can be improved.

In some examples, the increase rate coefficient of the target node may be obtained based on the difference between the target physiological parameter acquired by measurement and the target physiological parameter acquired by calculation located at the target node, and the increase rate coefficients of the other nodes. In this case, the increase rate coefficient of the target node can be calculated by using some of parameters acquired in a previous time nodes, thereby adaptively adjusting the increase rate coefficient, so that the accuracy of the sensitivity coefficient can be improved.

Hereinafter the acquisition way of the increase rate coefficient is further explained. In some examples, the increase rate coefficient may satisfy the formula:

$$
\begin{aligned}
\hat{\alpha}(k) &= G(k) - \hat{G}(k) + \hat{\alpha}(k-1) \\
&= e(k) + \hat{\alpha}(k-1)
\end{aligned}
,
$$

Wherein $\hat{\alpha}(k)$ may represent a predicted value of the increase rate coefficient at the $k^{th}$ time node, $\hat{G}(k)$ may represent a calculated value of the blood glucose concentration at the $k^{th}$ time node (i.e., a predicted value of the blood glucose concentration), and $\hat{e}(k)$ may represent a difference between the predicted value of the blood glucose concentration at the $k^{th}$ time node and a measured value of the blood glucose concentration.

It should be noted that the predicted value of the blood glucose concentration and the measured value of the blood glucose concentration may be understood in the following way: the predicted value $\hat{G}(k)$ of the blood glucose concentration is an intermediate parameter appearing in step S109, which can be obtained based on the blood glucose concentration at the k-1$^{th}$ time node, the predicted value of the insulin sensitivity coefficient, the active insulin, the increase rate coefficient and the blood glucose dynamics model, and the measured value of the blood glucose concentration is the blood glucose concentration obtained by the continuous blood glucose monitoring apparatus 10a or blood glucose meter 10b described above. Unless otherwise specified, the blood glucose concentration in the body of the object 2 and the blood glucose concentration involved in a derivation process in other steps may refer to the measured value of the blood glucose concentration.

In some examples, referring to simplified results of the formula that the increase rate coefficient satisfies. The increase rate coefficient may satisfy a second iteration formula:

$$
\hat{\alpha}(k) = e(k) + \hat{\alpha}(k-1)
,
$$

In other words, the increase rate coefficient may represent the sum of the differences between the predicted value of the blood glucose concentration and the measured value of the blood glucose concentration in different control steps (i.e., in different time nodes).

In some examples, the initial value of the increase rate coefficient may be 0, but the present disclosure is not limited to this. The increase rate coefficient may also be any value.

(Step S109)

In some examples, in step S109, the safe dose and the basal dose of the chemical substance can be calculated based on the preset parameters, the target physiological parameter, the residual activity parameter, the sensitivity coefficient and increase rate coefficient. The safe dose may refer to a dose that the target physiological parameter will not be lower than the safety threshold value in a long time after the chemical substance is infused into the object 2. The basal dose may refer to a dose that the target physiological parameter can reach a target value in a short time (such as one control step) after the chemical substance is infused into the object 2. In this case, after the infusion of the chemical substance, there will be the chemical substance remaining active at each time node in a metabolic process of the chemical substance. Under the action of the chemical substance remaining active at a plurality of time nodes, the adjustment of the target physiological parameter will overlap, which may cause the target physiological parameter to exceed the safety range (for example, below the safety threshold value), while the safe dose and the basal dose are calculated at the same time, so that a difference between two kinds of doses can be determined, which can then guarantee the safety and adjustment effect of the infusion dose.

In some examples, in a process of calculating the basal dose, according to the blood glucose dynamics model, the residual activity parameter of the chemical substance may satisfy the formula:

$$I_{eff}(k) = \frac{(G_d(k+1) - G(k) - \alpha(k))}{\varphi(k)}$$

Wherein $G_d(k+1)$ may represent the target value of the target physiological parameter at the $k+1^{th}$ time node. In some examples, the target value may be obtained via calculation. For example, the target value may be obtained through calculation of the target physiological parameter at the $k^{th}$ time node and the control step size. In some examples, the target value may also be obtained through manual input.

In some examples, in a process of calculating the basal dose, according to the blood glucose dynamics model, the residual activity parameter of the chemical substance may also satisfy the formula:

$$I_{eff}(k) = \frac{\rho(G_d(k+1) - G(k) - \alpha(k))}{\lambda + \varphi(k)}$$

Wherein $\lambda$, $\rho$ may represent adjustment parameters. In this case, since there may be a possibility that $\varphi$ approaches to 0, resulting in a large degree of change of $I_{eff}(k)$. $I_{eff}(k)$ can be stabilized by addition of the adjustment parameters, so that the subsequent calculated basal dose can be controlled within an appropriate range. In some examples, the adjustment of parameters can be selected or adjusted empirically.

In some examples, the basal dose may be obtained according to the metabolic model of the insulin and the calculation formula of the residual activity parameter of the chemical substance.

In some examples, in some examples, the adjustment parameters $\rho$ may be used to adjust the basal dose. For example, in a case where the adaptive closed-loop control method is used to adjust the blood glucose concentration, identical infusion dose may have different influence levels on adults and children. A safe and appropriate dose for the adults may have significant impact on the children (for example, causing hypoglycemia). Therefore, the dose for the children may be reduced in a certain proportion, so that a value range of $\rho$ may be set to be from 0 to 1, and different $\rho$ is set as to different populations. In this case, adaptability of the adaptive closed-loop control method to different populations can be improved.

In some examples, in a process of calculating the safe dose, the continuous effect of the chemical substance remaining active on the target physiological parameter may be calculated first. Taking the insulin as an example, the change of future active insulin volume over time may be predicted based on the metabolic model of the insulin and historical insulin infusion information.

In some examples, the future active insulin and the insulin sensitivity coefficient may be used to obtain the total impact of the insulin infused into the object 2 on the blood glucose concentration. Specifically, after the insulin is infused into the object 2 at the $k-1^{th}$ time node, the impact of active insulin on the blood glucose concentration may be integrated to obtain the total impact on the blood glucose concentration.

In some examples, when the total impact of insulin infusion at the $k-1^{th}$ time node on the blood glucose concentration, the sensitivity coefficient may be a constant. In this case, since one control step corresponds to one time node, each control step can calculate the total impact of the active insulin on the blood glucose concentration, and therefore, the blood glucose dynamics model established in a single control step may be appropriately simplified, while the total impact of the active insulin on the blood glucose concentration is calculated in one control step, the sensitivity coefficient is a constant, which can reduce the calculation cost and increase the calculation speed.

In some examples, in a process of calculating the safe dose, the safe dose may be obtained according to the total effect of the active insulin on the blood glucose concentration and the blood glucose dynamics model, without considering the increase rate coefficient.

In some examples, in a process of calculating the safe dose, PID (proportional integral derivative) control principle may also be added in a case where the increase rate coefficient is considered. Specifically, the increase rate coefficient may be used to construct a PID term. In this case, the accuracy and stability of the adaptive closed-loop control method can be improved.

(Step S111)

In some examples, in step S111, the target dose of the chemical substance may be calculated based on the safe dose and the basal dose. In this case, calculation results of the safe dose and basal dose may be considered at the same time and the appropriate target dose may be obtained.

In some examples, the smaller one in the safe dose and the basal dose may be taken as the target dose. In this case, the safety of the adaptive closed-loop control method can be improved.

In some examples, as described above, the chemical substance of the infusion dose can be allowed to enter the object 2 by means of infusion. The infusion dose is related to the target dose and the infusion accuracy. In some examples, rounding down may be used to calculated the infusion dose. For example, in the infusing device 20, the infusion accuracy is 0.1 U/time, and the calculated target dose is 6.47 U, the infusion dose may be 6.4 U. In this case, the calculated target dose can cooperate with actual infusion accuracy of an actual device to obtain the infusion dose that can be infused to the object 2 within an accuracy range.

In some examples, the infusion dose may be obtained through metering with the infusing device 20. In this case, due to uncertainty of actual infusion, metering of the infusing device 20 is used to obtain the infusion dose, capable of obtaining the actual dose of the chemical substance infused into the object 2 more accurately, facilitating to improve the accuracy of subsequent regulation.

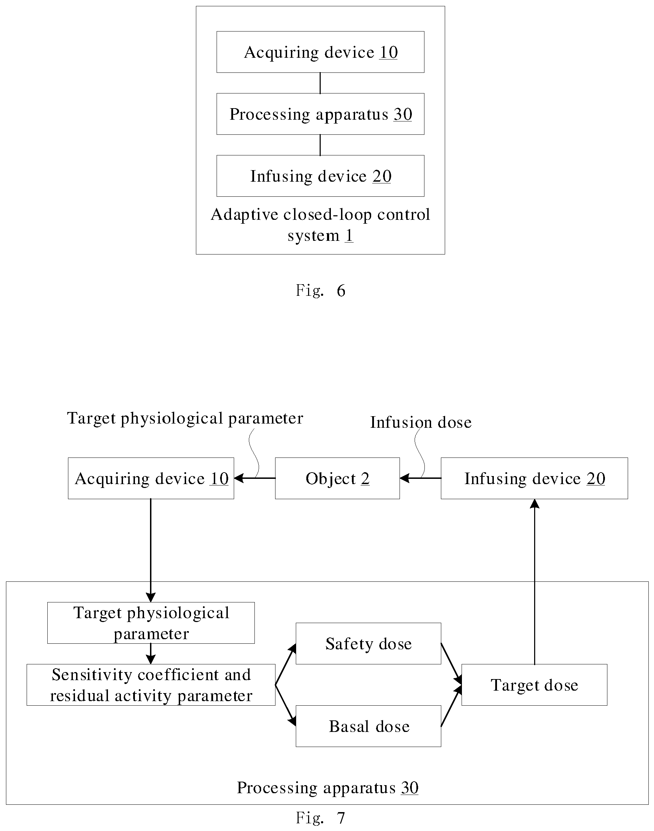

Fig. 6 is a structural block diagram illustrating a linear model-based adaptive closed-loop control system 1 involved in an embodiment of the present disclosure. Fig. 7 is a flow chart illustrating a linear model-based adaptive closed-loop control system 1 involved in an embodiment of the present disclosure.

As described above, the present disclosure further relates to a linear model-based adaptive closed-loop control system 1, using the adaptive closed-loop control method involved in an embodiment of the present disclosure to control a target physiological parameter of an object 2. In this case, a physiological parameter related to the target physiological parameter can be adaptively adjusted, the stability of the target physiological parameter of the object 2 can be improved, and input parameters can be reduced.

In some examples, referring to Fig. 6, the linear model-based adaptive closed-loop control system 1 may include an infusing device 20, a processing apparatus 30, and an acquiring device 10.

In some examples, referring to Fig. 7, the acquiring device 10 may be used to acquire the target physiological parameter of the object 2.

In some examples, referring to Fig. 7, the infusing device 20 may infuse the chemical substance into the object 2 to adjust the target physiological parameter of the object 2.

In some examples, referring to Fig. 7, the processing apparatus 30 may calculate the dose of the chemical substance required to be infused by the infusing device 20, based on preset coefficients input earlier and the target physiological parameter acquired by the acquiring device 10.

In some examples, referring to Fig. 7, after acquiring the target physiological parameter, the processing apparatus 30 may calculate the sensitivity coefficient and the residual activity parameter, calculate the safe dose and basal dose based on the sensitivity coefficient and the residual activity parameter, calculate the target dose based on the safe dose and the basal dose, and send the target dose to the infusing device 20.

In some examples, referring to Fig. 7, the infusing device 20 may infuse the chemical substance of infusion dose to the object 2 based on the target dose.

In some examples, the processing apparatus 30 may use the adaptive closed-loop control method involved in the embodiment of the present disclosure to obtain the target dose, and control the infusing device 20 to infuse the chemical substance.

(Simulation Results)

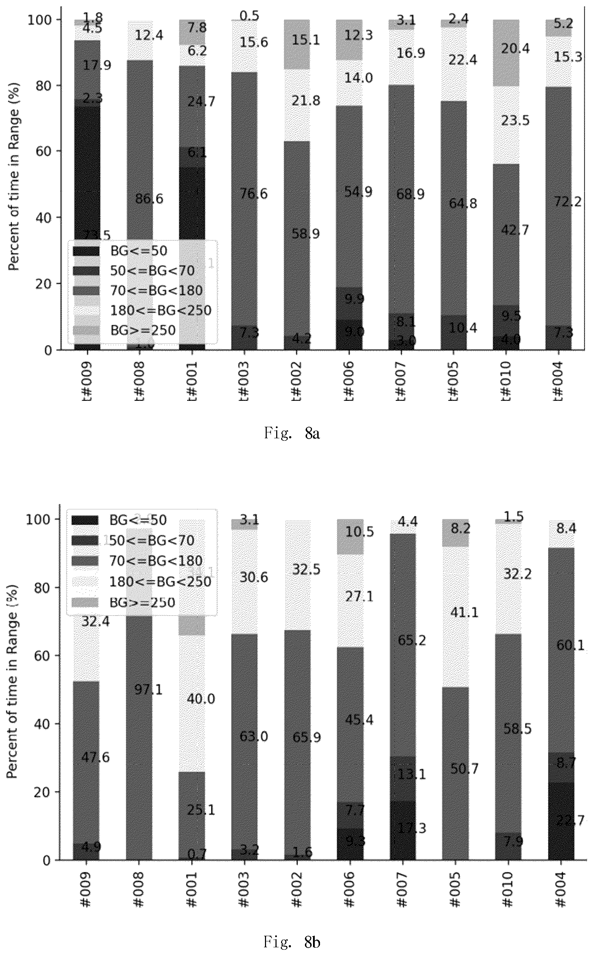

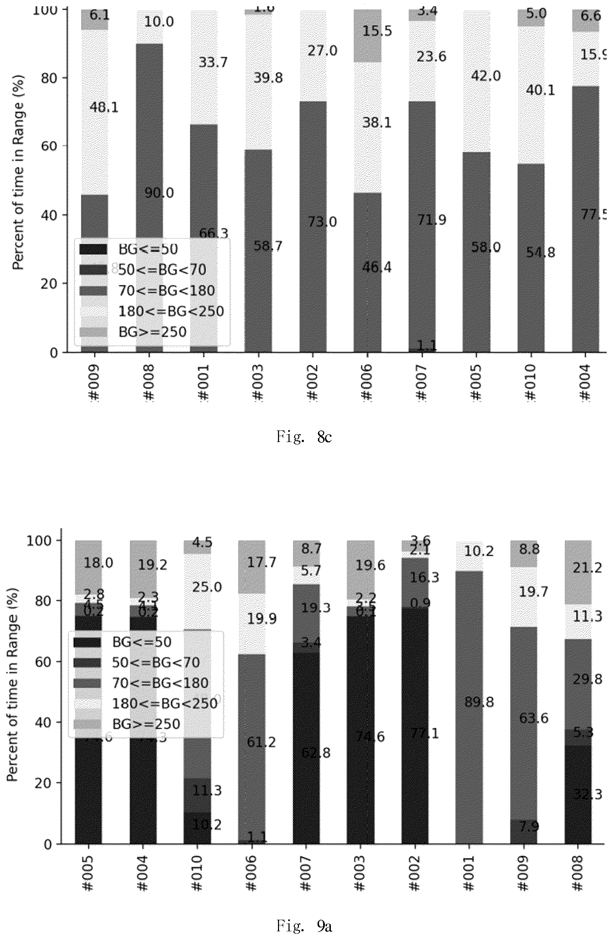

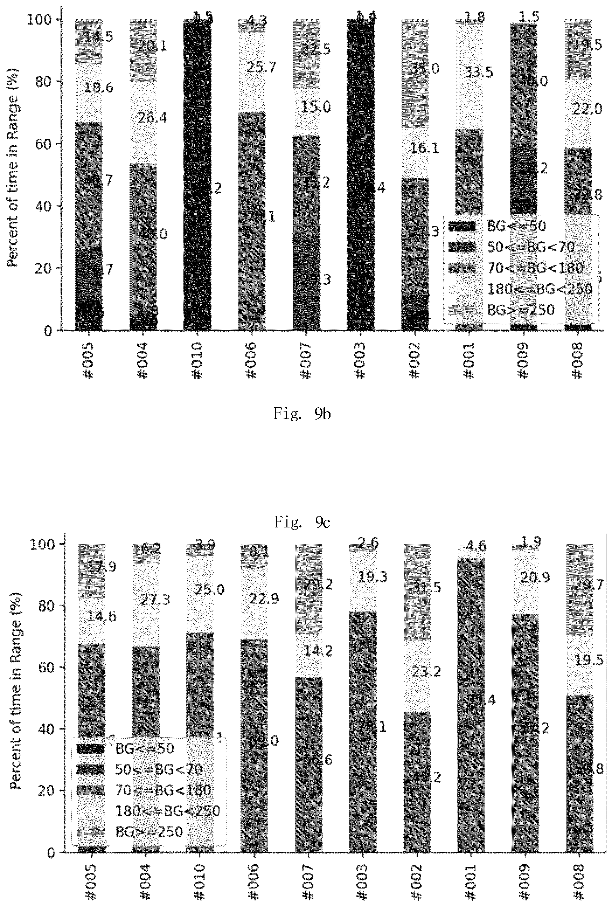

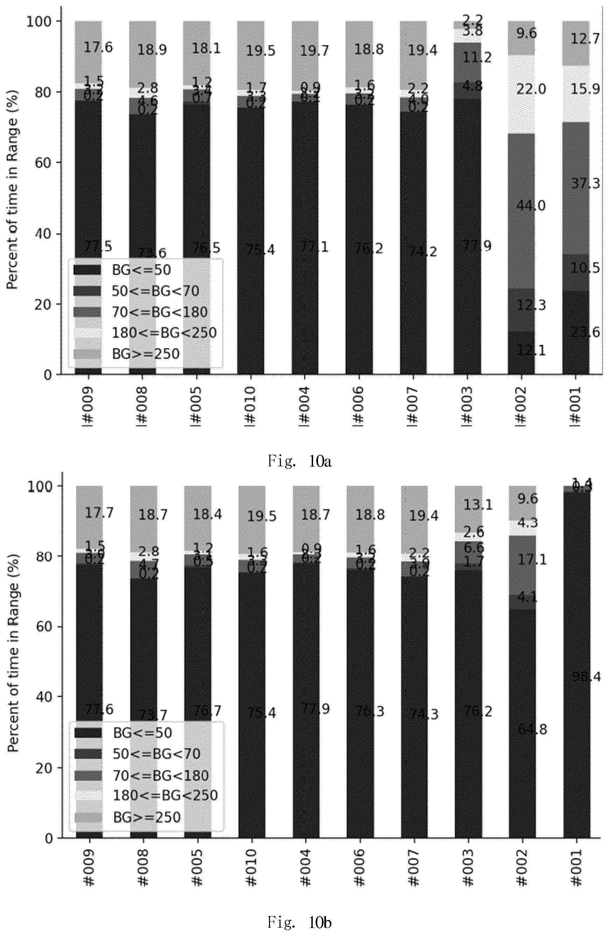

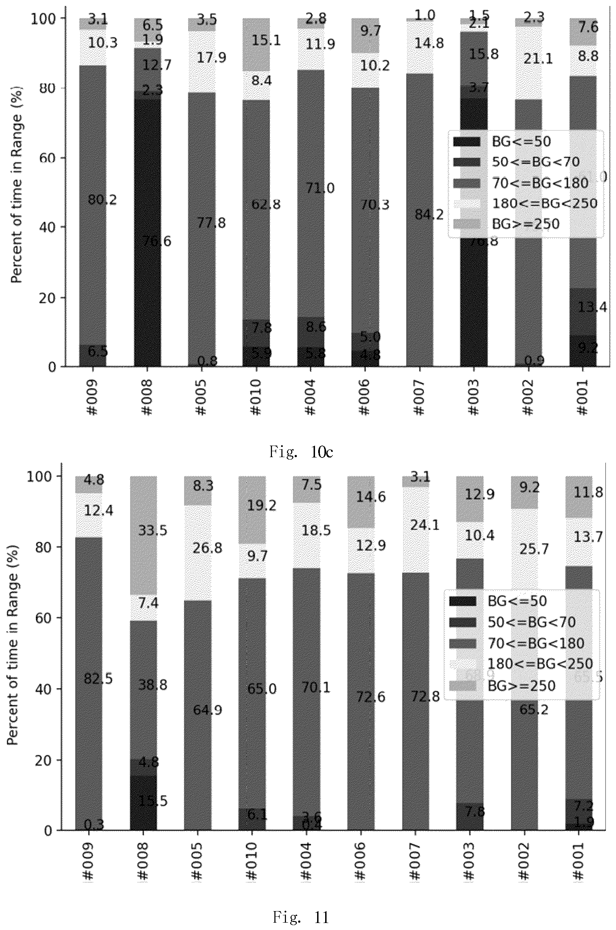

Fig. 8a is a simulation schematic diagram illustrating that a plain PID method is applied to an adult involved in an embodiment of the present disclosure. Fig. 8b is a simulation schematic diagram illustrating that a PIDIFB method is applied to an adult involved in an embodiment of the present disclosure. Fig. 8c is a simulation schematic diagram illustrating that an adaptive closed-loop control method is applied to an adult involved in an embodiment of the present disclosure. Fig. 9a is a simulation schematic diagram illustrating that a plain PID method is applied to an adolescent involved in an embodiment of the present disclosure. Fig. 9b is a simulation schematic diagram illustrating that a PIDIFB method is applied to an adolescent involved in an embodiment of the present disclosure. Fig. 9c is a simulation schematic diagram illustrating that an adaptive closed-loop control method is applied to an adolescent involved in an embodiment of the present disclosure. Fig. 10a is a simulation schematic diagram illustrating that a plain PID method is applied to a child involved in an embodiment of the present disclosure. Fig. 10b is a simulation schematic diagram illustrating that a PIDIFB method is applied to a child involved in an embodiment of the present disclosure. Fig. 10c is a simulation schematic diagram illustrating that an adaptive closed-loop control method is applied to a child involved in an embodiment of the present disclosure.

The simulation schematic diagrams are diagrams obtained through a Simglucose model. Simulation objects include adults, adolescents and children. Dexcom's and Insulet's continuous glucose monitoring apparatuses 10a (CGM) are used in the simulation. The initial value of the insulin sensitivity coefficient may be set by rule 1800.

In some examples, the plain PID method may refer to a control method that takes historical blood glucose data and insulin injection data as input, without stating the carbohydrate, and simply uses PID. The PIDIFB method may be a control method combining the PID method with insulin feedback. LMAPID may refer to the adaptive closed-loop control method involved in the embodiments of the present disclosure.

Referring to Fig. 8a to Fig. 10c, whether adults, adolescents or children, compared with the plain PID method and the PIDIFB method, the adaptive closed-loop control method involved in the embodiment of the present disclosure has better simulation results.

Referring to Fig. 10c, when the adaptive closed-loop control method is applied to the children, there is a risk of hypoglycemia in the simulation results of the objects 2 numbered # 008 and # 003. In this regard, the adaptive closed-loop control method may be optimized. For example, the target dose of the insulin may be reduced in an equal proportion for the children. Specifically, the target dose may be reduced by 50% to 90% in an equal proportion. In this case, the risk of hypoglycemia can be lowered.

Fig. 11 is a simulation schematic diagram illustrating that an optimized adaptive closed-loop control method is applied to a child involved in an embodiment of the present disclosure.

Referring to Fig. 11, the optimized adaptive closed-loop control method (the target dose is reduced by 70% in an equal proportion) effectively lowers the risk of hypoglycemia.

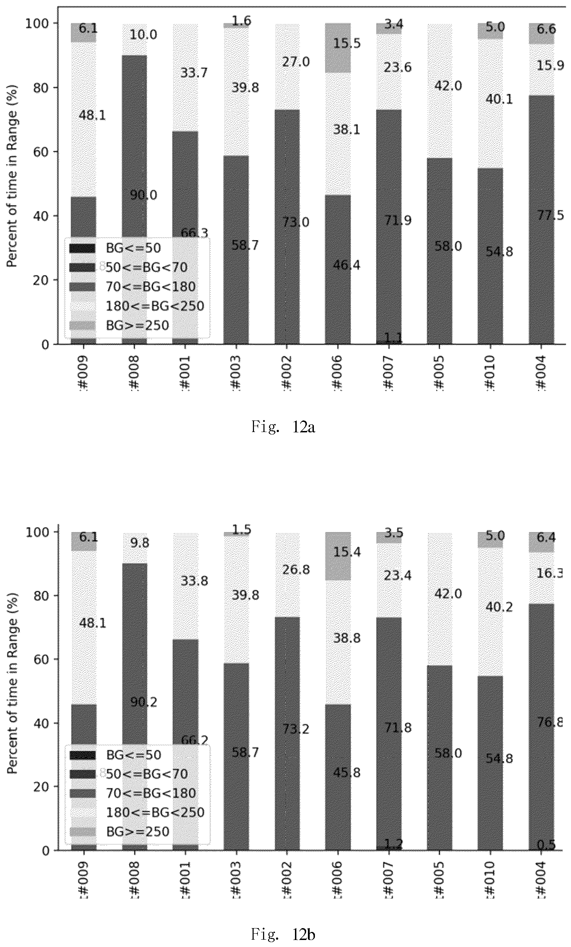

Fig. 12a is a simulation schematic diagram illustrating that an adaptive closed-loop control method with infinite infusion accuracy is applied to an adult involved in an embodiment of the present disclosure. Fig. 12b is a simulation schematic diagram illustrating that an adaptive closed-loop control method with finite infusion accuracy is applied to an adult involved in an embodiment of the present disclosure.

It should be noted that in the adaptive closed-loop control method with the infinite infusion accuracy, the target dose may be the same as the infusion dose. In the adaptive closed-loop control method with the finite infusion accuracy, the method described above based on the infusion accuracy and the target dose may be used to calculate the infusion dose. Fig. 12b represents that the infusion accuracy is 0.1 U/time. Referring to Fig. 12a and Fig. 12b, the simulation results of the adaptive closed-loop control method with the infinite infusion accuracy are roughly the same as those of the adaptive closed-loop control method with infusion accuracy of 0.1 U/time, which indicates that the adaptive closed-loop control method involved in the embodiments of the present disclosure is less sensitive to the infusion accuracy within a certain accuracy range. In other words, within the certain accuracy range, even if the adaptive closed-loop control method involved in the embodiments of the present disclosure is used in the infusing device 20 with relatively low accuracy, a relatively good control effect still can be kept.

While the present disclosure has been specifically described above in combination with the drawings and embodiments, it is understood that the above description does not limit the present disclosure in any form. Those skilled in the art may, as required, make changes to the present disclosure without departing from the essential spirit and scope of the present disclosure, and these changes fall within the scope of the present disclosure.

**Claims**

1. A linear model-based adaptive closed-loop control method, which is a control method to control a target physiological parameter of an object using a chemical substance, **characterized by** comprising:

   acquiring preset parameters,
   acquiring a target physiological parameter of the object and a residual activity parameter of the chemical substance,
   calculating a sensitivity coefficient of the chemical substance based on the target physiological parameter and the residual activity parameter, the sensitivity coefficient being used for characterizing an influence level of the chemical substance on the target physiological parameter,
   calculating increase a rate coefficient of the target physiological parameter based on the target physiological parameter and the residual activity parameter, the increase rate coefficient being used for characterizing an influence level of factors other than the chemical substance on the target physiological parameter,
   calculating a safe dose and a basal dose of the chemical substance based on the preset parameters, the target physiological parameter, the residual activity parameter, the sensitivity coefficient and the increase rate coefficient, and
   calculating a target dose of the chemical substance based on the safe dose and the basal dose.

2. The adaptive closed-loop control method according to claim 1, **characterized in that**
   the preset parameters include a physiological information of the object, a type of the chemical substance, a control step size, an unit of the target physiological parameter, a safety threshold value of the target physiological parameter, and an infusion accuracy of the chemical substance.

3. The adaptive closed-loop control method according to claim 1, **characterized in that** the preset parameters are obtained by means of data import or manual input.

4. The adaptive closed-loop control method according to claim 1, **characterized in that** the chemical substance of the infusion dose is enabled to enter the object by means of infusion, the infusion dose being related to the target dose and the infusion accuracy.

5. The adaptive closed-loop control method according to claim 4, **characterized in that** the infusion dose is obtained by metering of an infusing device.

6. The adaptive closed-loop control method according to claim 1, **characterized in that** the target physiological parameter is collected by a continuous blood glucose monitoring apparatus or a blood glucose meter.

7. The adaptive closed-loop control method according to claim 1, **characterized in that** the target physiological parameters of the object at a plurality of time nodes and the residual activity parameter of the chemical substance are acquired, the plurality of time nodes include a target node and other nodes, and the residual activity parameter of the target node is obtained based on the residual activity parameters of the other nodes.

8. The adaptive closed-loop control method according to claim 7, **characterized in that** the sensitivity coefficient located at the target node is obtained by minimizing a loss function, the loss function includes an error term and a regularization term, the error term is used for characterizing a difference between the target physiological parameter acquired by measurement and the target physiological parameter acquired by calculation, and the regularization term is used for characterizing a difference degree of the sensitivity coefficients of adjacent time nodes.

9. The adaptive closed-loop control method according to claim 8, **characterized in that** the sensitivity coefficient of the target node is acquired based on the sensitivity coefficients of the other nodes, the target physiological parameters of the other nodes, the residual activity parameters of the other nodes, and the target physiological parameter of the target node.

10. The adaptive closed-loop control method according to claim 7, **characterized in that** the increase rate coefficient of the target node is obtained based on the difference between the target physiological parameter acquired by measurement and the target physiological parameter acquired by calculation located at the target node, and the increase rate coefficients of the other nodes.

Fig. 1

Fig. 2

| Acquire preset parameters | S101 |

↓

| Acquire a target physiological parameter of an object and a residual activity parameter of a chemical substance | S103 |

↓

| Calculate a sensitivity coefficient of the chemical substance based on the target physiological parameter and the residual activity parameter | S105 |

↓

| Calculate an increase rate coefficient of the target physiological parameter based on the target physiological parameter and the residual activity parameter | S107 |

↓

| Calculate a safety dose and a basal dose of the chemical substance based on the preset parameters, the target physiological parameter, the residual activity parameter, the sensitivity coefficient and the increase rate coefficient | S109 |

↓

| Calculate a target dose of the chemical substance based on the safety dose and the basal dose | S111 |

Fig. 3

Fig. 4

Blood glucose concentration (mmol/L)

Time

Fig. 5

Acquiring device 10

Processing apparatus 30

Infusing device 20

Adaptive closed-loop control system 1

Fig. 6

Target physiological parameter

Infusion dose

Acquiring device 10 ← Object 2 ← Infusing device 20

Target physiological parameter

Sensitivity coefficient and residual activity parameter

Safety dose

Basal dose

Target dose

Processing apparatus 30

Fig. 7

Fig. 8a

Fig. 8b

Fig. 8c

Fig. 9a

Fig. 9b

Fig. 9c

Fig. 10a

Fig. 10b

Fig. 10c

Fig. 11

Fig. 12a

Fig. 12b

**INTERNATIONAL SEARCH REPORT**

| International application No. |
|---|
| **PCT/CN2023/099003** |

| A. | CLASSIFICATION OF SUBJECT MATTER |
|---|---|
| | A61M5/168(2006.01)i; G16H20/17(2018.01)i; A61M5/172(2006.01)i |

According to International Patent Classification (IPC) or to both national classification and IPC

| B. | FIELDS SEARCHED |
|---|---|

Minimum documentation searched (classification system followed by classification symbols)

IPC: A61M5/-; G16H20/-; A61M5/-

Documentation searched other than minimum documentation to the extent that such documents are included in the fields searched

Electronic data base consulted during the international search (name of data base and, where practicable, search terms used)

DWPI, WPABS, CNTXT, ENTXT, CNKI: 山东硅基算力, 闭环, 控制, 自适应, 胰岛素, 血糖, 活性, 余量, 残留, 剩余, 参数, 增速, 敏感, 目标, 剂量, 节点, 损失, close w loop, control+, parameter, glucose, blood sugar, activity, speed increas+, sensitivity, coefficient, dose

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115645679 A (SHANDONG SILICON-BASED COMPUTING TECHNOLOGY CO., LTD.) 31 January 2023 (2023-01-31) claims 1-10, description, paragraphs [0039]-[0166], and figures 1-12b | 1-10 |
| A | CN 114144665 A (COMPANION MEDICAL INC.) 04 March 2022 (2022-03-04) description, paragraphs [0064]-[0149], and figures 1-6 | 1-10 |
| A | CN 106659843 A (BIGFOOT BIOMEDICAL INC.) 10 May 2017 (2017-05-10) entire document | 1-10 |
| A | CN 110869075 A (LIFESCAN IP HOLDINGS, LLC) 06 March 2020 (2020-03-06) entire document | 1-10 |
| A | CN 101871930 A (F. HOFFMANN-LA ROCHE AG) 27 October 2010 (2010-10-27) entire document | 1-10 |
| A | CN 105339943 A (FRESENIUS VIAL SAS) 17 February 2016 (2016-02-17) entire document | 1-10 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **18 July 2023** | **24 July 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/099003** |

### C. DOCUMENTS CONSIDERED TO BE RELEVANT

| Category* | Citation of document, with indication, where appropriate, of the relevant passages | Relevant to claim No. |
| --- | --- | --- |
| A | CN 112133439 A (BEIJING INSTITUTE OF TECHNOLOGY) 25 December 2020 (2020-12-25)<br>    entire document | 1-10 |
| A | KR 20200119216 A (POSTECH RESEARCH AND BUSINESS DEVELOPMENT FOUNDATION) 19 October 2020 (2020-10-19)<br>    entire document | 1-10 |

Form PCT/ISA/210 (second sheet) (July 2022)

## INTERNATIONAL SEARCH REPORT
### Information on patent family members

International application No.

**PCT/CN2023/099003**

| Patent document cited in search report | | | Publication date (day/month/year) | Patent family member(s) | | | Publication date (day/month/year) |
|---|---|---|---|---|---|---|---|
| CN | 115645679 | A | 31 January 2023 | None | | | |
| CN | 114144665 | A | 04 March 2022 | WO | 2020231866 | A1 | 19 November 2020 |
| | | | | US | 2022211944 | A1 | 07 July 2022 |
| | | | | EP | 3966560 | A1 | 16 March 2022 |
| CN | 106659843 | A | 10 May 2017 | US | 2017189615 | A1 | 06 July 2017 |
| | | | | US | 10549037 | B2 | 04 February 2020 |
| | | | | WO | 2016004210 | A1 | 07 January 2016 |
| | | | | US | 2016000998 | A1 | 07 January 2016 |
| | | | | US | 9629901 | B2 | 25 April 2017 |
| | | | | US | 2020171242 | A1 | 04 June 2020 |
| | | | | EP | 3164170 | A1 | 10 May 2017 |
| | | | | EP | 3164170 | B1 | 08 September 2021 |
| | | | | CN | 106659843 | B | 28 August 2020 |
| CN | 110869075 | A | 06 March 2020 | KR | 20190132684 | A | 28 November 2019 |
| | | | | US | 2018289891 | A1 | 11 October 2018 |
| | | | | US | 10729849 | B2 | 04 August 2020 |
| | | | | EP | 3606584 | A1 | 12 February 2020 |
| | | | | WO | 2018187539 | A1 | 11 October 2018 |
| | | | | CN | 110869075 | B | 18 March 2022 |
| CN | 101871930 | A | 27 October 2010 | JP | 2010256356 | A | 11 November 2010 |
| | | | | JP | 5587022 | B2 | 10 September 2014 |
| | | | | US | 2010271213 | A1 | 28 October 2010 |
| | | | | EP | 2243423 | A1 | 27 October 2010 |
| | | | | EP | 2243423 | B1 | 02 May 2012 |
| CN | 105339943 | A | 17 February 2016 | WO | 2014202233 | A1 | 24 December 2014 |
| | | | | EP | 3011487 | A1 | 27 April 2016 |
| | | | | US | 2016089494 | A1 | 31 March 2016 |
| | | | | US | 10525199 | B2 | 07 January 2020 |
| | | | | JP | 2016530905 | A | 06 October 2016 |
| | | | | CN | 105339943 | B | 01 January 2019 |
| CN | 112133439 | A | 25 December 2020 | CN | 112133439 | B | 03 June 2022 |
| KR | 20200119216 | A | 19 October 2020 | KR | 102464709 | B1 | 09 November 2022 |

Form PCT/ISA/210 (patent family annex) (July 2022)

**REFERENCES CITED IN THE DESCRIPTION**

**Patent documents cited in the description**

- CN 113453619 A